# EUROPEAN PATENT APPLICATION

(11) **EP 4 438 589 A1**
(43) Date of publication of application: **02.10.2024**
(21) Application number: 22898950.5
(22) Date of filing: 18.11.2022
(51) Int. Cl.: C07C 68/06, C07C 69/96

(54) **METHOD FOR PRODUCING CARBONATE**

(30) Priority: 25.11.2021 KR 20210164915
(71) Applicant: Lotte Chemical Corporation, Seoul, 05551 (KR)
(72) Inventor: KIM, Wang Gyu, Daejeon 34110 (KR); KIM, Jin Hyung, Daejeon 34110 (KR); BAEK, Mi Hwa, Daejeon 34110 (KR); HAN, Eun Hye, Daejeon 34110 (KR)
(74) Representative: Germain Maureau
(86) International application number: PCT/KR2022/018254
(87) International publication number: WO 2023/096268

(57) **Abstract**

The present invention relates to a method for preparing a carbonate. According to the method according to the present invention, it is possible to increase the DMC conversion rate and EMC selectivity in a transesterification reaction of DMC and EtOH.

## Description

### [Technical Field]

The present invention relates to a method for preparing a carbonate.

### [Background Art]

As an organic solvent for a battery electrolyte, ethyl methyl carbonate (hereinafter, "EMC") and diethyl carbonate (hereinafter, "DEC") are mainly used. EMC and DEC are prepared through a transesterification reaction using dimethyl carbonate (hereinafter "DMC") and ethanol (hereinafter "EtOH") as raw materials. The transesterification reaction is also a reversible reaction. In addition, the transesterification reaction may be performed in the presence of a catalyst. As the catalyst, a basic catalyst may be mainly used, and a representative example thereof is sodium methoxide (hereinafter, "SME"). The reaction formula was summarized in Reaction Formula 1 below:

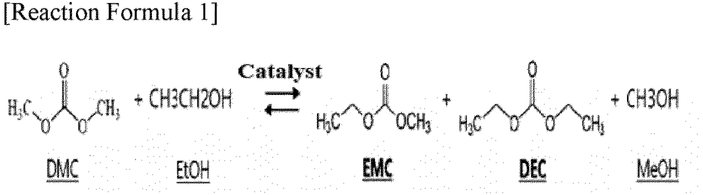

According to market prediction data of EMC and DMC, a future demand for EMC is approximately 5 times higher than for DEC. Therefore, it is necessary to prepare EMC with high selectivity using the same raw materials.

The prior arts (e.g., "Patent Document 1" and "Patent Document 2") prepare DMC and EMC using a reactive distillation device. In the prior arts, the reaction may proceed with high DMC conversion rate. However, in the prior arts, as the DMC conversion rate increases, the selectivity for DEC instead of EMC increases. Accordingly, it is difficult to increase the selectivity of EMC using the prior arts. In addition, in the prior arts, an operation method is very difficult.

Therefore, research and development on methods capable of increasing the selectivity of EMC in the reaction are required.

### [Prior Arts]

### [Patent Documents]

(Patent Document 1) CN 103804124B
(Patent Document 2) KR 10-1668571B

### [Disclosure]

### [Technical Problem]

An object of the present invention is to simultaneously increase the DMC conversion rate and EMC selectivity in a transesterification reaction of DMC and EtOH.

### [Technical Solution]

An aspect of the present invention provides a method for preparing a carbonate including: a first step of preparing a raw material mixture containing dimethyl carbonate and ethanol; and a second step of preparing a product containing ethyl methyl carbonate and diethyl carbonate by inducing a transesterification reaction in the raw material mixture, in which in the first step, diethyl carbonate is further included in the raw material mixture.

### [Advantageous Effects]

According to the method of the present invention, it is possible to increase the DMC conversion rate and EMC selectivity in a transesterification reaction of DMC and EtOH.

### [Description of Drawings]

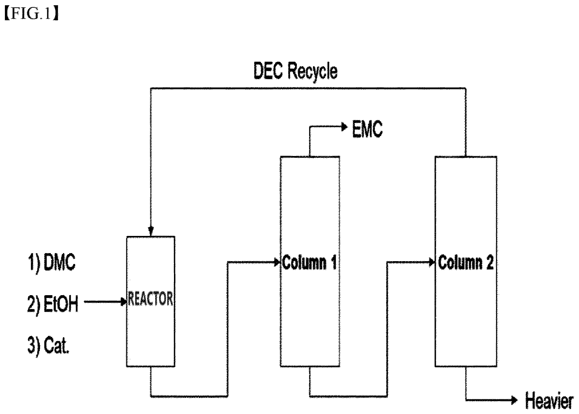

FIG. 1 illustrates a method of an exemplary embodiment of the present invention for further including DEC in a raw material mixture.

### [Best Mode of the Invention]

Hereinafter, the contents of the present invention will be described in more detail.

The present invention relates to a method for preparing a carbonate. Specifically, the present invention relates to a method for preparing a mixture containing EMC and DEC as carbonates. More specifically, the present invention relates to a method for improving the selectivity of EMC in the mixture. Hereinafter, the method for preparing the carbonate of the present invention may be referred to as the "method of the present invention".

In this specification, the "selectivity" means a ratio of a target product to the total products in a chemical reaction in which a plurality of products are produced.

In the present invention, among EMC, DEC, and methanol (hereinafter, "MeOH"), which are products of the transesterification reaction of DMC and EtOH, EMC is used as the target product. In addition, in the present invention, the selectivity of EMC refers to a ratio of EMC to the total of EMC and DEC. In addition, in the present invention, the selectivity of DEC refers to a ratio of DEC to the total of EMC and DEC.

In the method of the present invention, the carbonate is prepared through at least two steps.

In the method of the present invention, in a first step, a raw material mixture containing DMC and EtOH is prepared. As described below, in the present invention, it is confirmed that the selectivity of EMC, the target product, is determined by the equilibrium of the chemical reaction of the raw materials, and that the equilibrium is controlled by the composition of the reactants in the raw material mixture.

In the method of the present invention, in the second step, a product containing EMC and DEC is prepared by inducing the transesterification reaction in the raw material mixture. The product may further include MeOH as shown in Reaction Formula 1 above. A battery electrolyte uses the EMC and DEC as solvents. Here, the EMC is a target product of the method of the present invention.

In the method of the present invention, in the first step, a non-target product is further included in the raw material mixture. By adding the non-target product to the raw materials, the production amount of the target product may be increased under the same conversion rate of reactants according to the Le Chatelier's principle. That is, in the first step of the present invention, the DEC is further included in the raw material mixture. Through this, the selectivity of EMC may be increased under the same conversion rate of reactants.

The method of further including DEC, the non-target product, in the raw material mixture is not particularly limited. As such a method, for example, recycling DEC from the product that has completed the second step to be supplied to the raw materials, separately providing DEC to be added to the raw materials, or the like may be considered.

That is, in an exemplary embodiment, in the first step, the DEC prepared in the second step may be further included in the raw material mixture. Specifically, in the first step, the diethyl carbonate prepared in the second step may be refluxed and further included in the raw material mixture.

FIG. 1 briefly illustrates the method therefor. The method of re-supplying DEC according to FIG. 1 will be described in more detail. First, DMC, EtOH, and a catalyst are added to the reactor and then reacted to produce EMC, DEC, and MeOH. A product stream generated above is transmitted to a first distillation column (Column 1). In the first distillation column, EMC, a low boiling point compound, may be separated from the column overhead. Then, the remaining product is transmitted to a second distillation column (Column 2). In the second column, a heavy component (Heavier) may be discharged from the column bottom, and DEC may be discharged from the column overhead. Subsequently, the DEC at the column overhead may be recycled or refluxed back into the reactor to further include DEC, a non-target product, in the raw material mixture. The process illustrated in FIG. 1 is exemplary, and in the actual process of performing the method of the present invention, other distillation columns for separating unreacted products and/or alcohols (methanol, ethanol, etc.) may be further included.

In addition, in another exemplary embodiment, the first step may include further including (different) DEC other than the DEC prepared in the second step, for example, DEC produced or prepared separately, in the raw material mixture.

As described above, the selectivity of the target product of the present invention is not significantly changed depending on the reaction temperature or the catalyst amount used in the present invention, but varies depending on the composition of the reactants included in the raw material mixture. In order to maximize the purpose of the present invention, that is, the conversion rate of DMC, a reactant, and the selectivity of EMC, a target product, the ratio of substances included in the raw materials of the present invention may also be appropriately adjusted.

In an exemplary embodiment, the ratio of the mole number (mol_{EtOH}) of ethanol in the raw material mixture to the mole number (mol_{DMC}) of dimethyl carbonate may be in the range of 0.2 to 10. In another exemplary embodiment, the lower limit of the ratio may be 0.3, 0.4, 0.5, 0.6, 0.7, 0.8, 0.9 or 1.0. In another exemplary embodiment, the upper limit of the ratio may be 9, 8, 7, 6, 5, 4, 3, 2 or 1.

In an exemplary embodiment, the ratio of DEC further included in the raw material mixture may also be appropriately adjusted. Meanwhile, since DEC directly affects the conversion rate of DMC as the reactant and the conversion rate of EMC as the target product, the amount of DEC added herein may be further adjusted when the ratio of DMC as the reactant to EtOH as another reactant is within a predetermined range.

In an exemplary embodiment, a ratio of the mole number (mol_{EtOH}) of ethanol in the raw material mixture to the mole number (mol_{DMC}) of dimethyl carbonate may be in the range of 0.9 to 1.1. At this time, a ratio (mol_{DEC}/mol_{DMC}) of the mole number (mol_{DEC}) of diethyl carbonate and the mole number (mol_{DMC}) of dimethyl carbonate may be in the range of 0.01 to 0.2. In another exemplary embodiment, the ratio may be in the range of 0.014 to 0.016. For reference, as described above, the mol ratio of DEC to DMC may vary depending on the ratio of EtOH in the raw materials to DMC.

In an exemplary embodiment, the second step, that is, the transesterification reaction of DMC and EtOH in the raw materials may be performed under specific conditions.

As described above, the raw materials applied in the method of the present invention include DMC and EtOH, and the reaction thereof may be performed in the presence of a catalyst. In an exemplary embodiment, the catalyst may be a basic catalyst. Specifically, the basic catalyst may include sodium methoxide, sodium hydroxide, sodium ethoxide, potassium hydroxide, potassium ethoxide, potassium methoxide, or a combination thereof. More specifically, the basic catalyst may be sodium methoxide.

In addition, in the present invention, the reaction may be performed in a specific reactor. A chemical reactor is largely divided into a batch reactor and a continuous flow reactor. Here, the continuous flow reactor is further divided into a continuously stirred tank reactor (CSTR) and a plug flow reactor (PFR). In reactions involving the catalyst, the PFR may serve as a packed bed reactor (PBR). In the present invention, the type of reactor in which the transesterification reaction is performed is not particularly limited. That is, the second step may also be performed in a batch reactor or a continuous reactor. In an exemplary embodiment, the second step may be performed in PBR or CSTR. Specifically, the second step may be performed in CSTR.

As described above, the catalyst content does not affect the selectivity of the target product of the present invention, but may be appropriately adjusted in terms of shortening the time to achieve the selectivity. In an exemplary embodiment, the used amount of the basic catalyst in the second step may be in the range of 0.02 parts by weight to 1 part by weight based on 100 parts by weight of dimethyl carbonate of the raw material mixture. In another exemplary embodiment, the lower limit of the used amount of the basic catalyst may be 0.05 parts by weight, 0.07 parts by weight, or 0.1 parts by weight. In another exemplary embodiment, the upper limit of the used amount of the basic catalyst may be 0.9 parts by weight, 0.8 parts by weight, 0.7 parts by weight, 0.6 parts by weight, 0.5 parts by weight, 0.4 parts by weight, 0.3 parts by weight, 0.2 parts by weight, or 0.1 parts by weight.

As described above, the temperature of the transesterification reaction does not affect the selectivity of the target product of the present invention, but may be appropriately adjusted in terms of shortening the time to achieve the selectivity. In an exemplary embodiment, the second step may be performed at a temperature in the range of 30°C to 100°C. In another exemplary embodiment, the lower limit of the temperature for the second step may be 35°C, 40°C, 45°C, or 50°C. In another exemplary embodiment, the upper limit of the temperature for the second step may be 90°C, 80°C, 70°C, 60°C, 55°C, or 50°C.

Hereinafter, the present invention will be described in more detail with reference to Examples. However, the scope of the present invention is not limited to the following Examples.

### [Experimental Example 1] Qualitative and quantitative analysis

Qualitative and quantitative analysis of a product was performed by gas chromatography (GC). Specifically, GC was performed on a sample in which 1 g of an analyte material was taken and mixed with 0.1 g of m-xylene. An YL6500GC product from YOUNGIN Chromas was used as GC equipment, in which a GC column was DB-1 30 m * 0.32 mm from Agilent and a GC detector was FID. The conversion rate of DMC was mol% of DMC consumption to DMC supply. The selectivity of EMC was mol% of the production of EMC to the total amount of EMC and DEC. The selectivity of DEC was mol% of the production of DEC to the total amount of EMC and DEC.

### [Experimental Example 2] Reaction of DMC and EtOH

The reaction illustrated in Reaction Formula 1 was performed in a 500 mL reactor. The contents of DMC, EtOH, and SME catalysts and the reactor temperature were adjusted as shown in Table 1 below. In addition, the selectivity of EMC and DEC produced accordingly was also shown in Table 1 below.

**[Table 1]**

| Classification | Reaction temperature (°C) | Catalyst added amount based on DMC (wt%) | EtOH/ DMC (mol ratio) | DMC added amount (g) | EtOH added amount (g) | DMC conversion rate (%) | EMC selectivity (%) | DEC selectivity (%) |
|---|---|---|---|---|---|---|---|---|
| EtOH/ DMC ratio | 50 | 0.1 | 0.2 | 90.1 | 9.2 | 21 | 96 | 4 |
| | 50 | 0.1 | 0.4 | 90.1 | 18.4 | 32 | 92 | 8 |
| | 50 | 0.1 | 0.5 | 90.1 | 23 | 36 | 90 | 10 |
| | 50 | 0.1 | 0.8 | 90.1 | 36.9 | 48 | 85 | 15 |
| | 50 | 0.1 | 1 | 90.1 | 46.1 | 54 | 82 | 18 |
| | 50 | 0.1 | 1.5 | 90.1 | 69.1 | 65 | 76 | 24 |
| | 50 | 0.1 | 2 | 90.1 | 92.1 | 72 | 68 | 32 |
| | 50 | 0.1 | 4 | 90.1 | 184.3 | 87 | 56 | 44 |
| | 50 | 0.1 | 8 | 90.1 | 368.6 | 94 | 47 | 53 |
| Temperature | 40 | 0.1 | 1 | 90.1 | 46.1 | 54 | 83 | 17 |
| | 60 | 0.1 | 1 | 90.1 | 46.1 | 54 | 82 | 18 |
| | 70 | 0.1 | 1 | 90.1 | 46.1 | 53 | 82 | 18 |
| Catalyst content | 50 | 0.3 | 1 | 90.1 | 46.1 | 54 | 82 | 18 |
| | 50 | 0.5 | 1 | 90.1 | 46.1 | 55 | 81 | 19 |
| | 50 | 0.7 | 1 | 90.1 | 46.1 | 54 | 82 | 18 |
| | 50 | 0.9 | 1 | 90.1 | 46.1 | 54 | 83 | 17 |

Through Table 1, it may be seen that in the transesterification reaction of DMC and EtOH, the selectivity of EMC varies greatly depending on a composition of the reaction raw materials, that is, a mol ratio of EtOH to DMC. In addition, through Table 1, it may be seen that in the transesterification reaction of DMC and EtOH, the selectivity of EMC does not change even if the catalyst concentration and reaction temperature are changed. As a result, it may be seen that in order to increase the selectivity of EMC, the target product, at least the composition of the raw material mixture needs to be changed.

### [Example 1]

90.08 g (1 mol) of DMC, 46.07 g (1 mol) of EtOH, and 1.7 g (0.0144 mol) of DEC were mixed in a 500 mL reactor. Next, the mixture was stirred at 500 rpm and added with 0.1 wt% of a NaOCH₃ catalyst based on the weight of DMC while the temperature was increased to 50°C. The reaction was performed for 1 hour. After the reaction was completed, a sample in the reactor was taken and the concentrations of DMC, EMC, and DEC were measured through GC analysis. As a result, the conversion rate of DMC was 53.1%, the EMC selectivity was 85%, and the DEC selectivity was 15%.

### [Example 2]

A reaction was performed in the same manner as in Example 1, except that the added amount of DEC was changed to 5 g (0.0423 mol). As a result, the conversion rate of DMC was 53.4%, the EMC selectivity was 87.9%, and the DEC selectivity was 12.1%.

### [Example 3]

A reaction was performed in the same manner as in Example 1, except that the added amount of DEC was changed to 7 g (0.0593 mol). As a result, the conversion rate of DMC was 53.7%, the EMC selectivity was 90.1%, and the DEC selectivity was 9.9%.

### [Example 4]

A reaction was performed in the same manner as in Example 1, except that the added amount of DEC was changed to 13 g (0.1100 mol). As a result, the conversion rate of DMC was 54.2%, the EMC selectivity was 95.4%, and the DEC selectivity was 4.6%.

### [Example 5]

A reaction was performed in the same manner as in Example 1, except that the added amount of DEC was changed to 17.8 g (0.1507 mol). As a result, the conversion rate of DMC was 54.7%, the EMC selectivity was 99.9%, and the DEC selectivity was 0.1%.

### [Comparative Example 1]

A reaction was performed in the same manner as in Example 1, except that DEC was not added to the raw materials. As a result, the conversion rate of DMC was 54%, the EMC selectivity was 82%, and the DEC selectivity was 18%.

### [Comparative Example 2]

A reaction was performed in the same manner as in Example 1, except that DEC was not added to the raw materials and the mol ratio (EtOH/DMC) of EtOH to DMC was changed to 0.2. As a result, the conversion rate of DMC was 21%, the EMC selectivity was 96%, and the DEC selectivity was 4%.

The information and results of Examples and Comparative Examples were summarized in Table 2 below.

**[Table 2]**

| Classification | Com. Ex. 1 | Com. Ex. 2 | Ex. 1 | Ex. 2 | Ex. 3 | Ex. 4 | Ex. 5 |
|---|---|---|---|---|---|---|---|
| EtOH/DMC (mol ratio) | 1 | 0.2 | 1 | 1 | 1 | 1 | 1 |
| DMC added amount (mol) | 1 | 1 | 1 | 1 | 1 | 1 | 1 |
| DMC added amount (g) | 90.10 | 90.10 | 90.1 | 90.10 | 90.10 | 90.10 | 90.10 |
| EtOH added amount (mol) | 1 | 0.2 | 1 | 1 | 1 | 1 | 1 |
| EtOH added amount (g) | 46.07 | 9.2 | 46.07 | 46.07 | 46.07 | 46.07 | 46.07 |
| DEC added amount (mol) | 0 | 0 | 0.0144 | 0.0423 | 0.0593 | 0.1100 | 0.1507 |
| DEC added amount (g) | 0 | 0 | 1.7 | 5 | 7 | 13 | 17.8 |
| DMC conversion rate (%) | 54 | 21 | 53.1 | 53.4 | 53.7 | 54.2 | 54.7 |
| EMC selectivity (%) | 82 | 96 | 85 | 87.9 | 90.1 | 95.4 | 99.9 |
| DEC selectivity (%) | 18 | 4 | 15 | 12.1 | 9.9 | 4.6 | 0.1 |

Through Table 2, it may be seen that the selectivity of EMC increases when DEC is added under the same conditions (Example 1 and Comparative Example 1). In addition, it may be seen that when increasing the EMC selectivity without adding DEC, the conversion rate is inevitably reduced (Example 4 and Comparative Example 2).

## Claims

1. A method for preparing a carbonate comprising:
a first step of preparing a raw material mixture containing dimethyl carbonate and ethanol; and
a second step of preparing a product containing ethyl methyl carbonate and diethyl carbonate by inducing a transesterification reaction in the raw material mixture,
wherein in the first step, diethyl carbonate is further included in the raw material mixture.

2. The method for preparing the carbonate of claim 1, wherein the second step is performed in a continuous stirred tank reactor.

3. The method for preparing the carbonate of claim 1, wherein the second step is performed in the presence of a basic catalyst including sodium methoxide, sodium hydroxide, sodium ethoxide, potassium hydroxide, potassium ethoxide, potassium methoxide, or a combination thereof.

4. The method for preparing the carbonate of claim 1, wherein a ratio of the mole number (mol_{EtOH}) of ethanol in the raw material mixture to the mole number (mol_{DMC}) of dimethyl carbonate is in the range of 0.2 to 10.

5. The method for preparing the carbonate of claim 3, wherein the used amount of the basic catalyst in the second step is in the range of 0.02 parts by weight to 1 part by weight based on 100 parts by weight of dimethyl carbonate of the raw material mixture.

6. The method for preparing the carbonate of claim 1, wherein the second step is performed at a temperature in the range of 30°C to 100°C.

7. The method for preparing the carbonate of claim 1, wherein a ratio of the mole number (mol_{EtOH}) of ethanol in the raw material mixture to the mole number (mol_{DMC}) of dimethyl carbonate is in the range of 0.9 to 1.1, and a ratio (mol_{DEC}/mol_{DMC}) of the mole number (mol_{DEC}) of diethyl carbonate to the mole number (mol_{DMC}) of dimethyl carbonate is in the range of 0.01 to 0.2.

8. The method for preparing the carbonate of claim 1, wherein in the first step, the diethyl carbonate prepared in the second step is refluxed and further included in the raw material mixture.

9. The method for preparing the carbonate of claim 1, wherein in the first step, diethyl carbonate other than the diethyl carbonate prepared in the second step is further included in the raw material mixture.
